(19) Europäisches Patentamt
European Patent Office
Office européen des brevets

(11) EP 0 771 187 B1

(12) # FASCICULE DE BREVET EUROPEEN

(45) Date de publication et mention de la délivrance du brevet:
**28.10.1998 Bulletin 1998/44**

(21) Numéro de dépôt: **95926403.7**

(22) Date de dépôt: **20.07.1995**

(51) Int. Cl.$^6$: **A61K 7/06**, A61K 7/48, A61K 7/04, A61K 31/44 // (A61K31/44, 31:165)

(86) Numéro de dépôt international:
**PCT/FR95/00974**

(87) Numéro de publication internationale:
**WO 96/02226 (01.02.1996 Gazette 1996/06)**

(54) **NOUVEAU PRODUIT DE COMBINAISON COMPRENANT UN AGENT ANTIFONGIQUE ET DU CROTAMITON COMME POTENTIALISATEUR DE L'ACTIVITE DE L'AGENT ANTIFONGIQUE, ET COMPOSITIONS DERMATOLOGIQUES ET/OU COSMETIQUES LE COMPRENANT**

DURCH KOMBINATION EINES ANTIMYKOTISCHEN MITTELS UND CROTAMITON ALS VERSTÄRKER FÜR ANTIMYKOTISCH EFFEKT ENTHALTENDES NEUES PRODUKT UND DIESES ENTHALTENDE KOSMETISCHE UND/ODER DERMATOLOGISCHE ZUSAMMENSETZUNG

NOVEL COMBINATION PRODUCT COMPRISING AN ANTIFUNGAL AGENT AND CROTAMITON AS AN ANTIFUNGAL ACTIVITY ENHANCER AND DERMATOLOGICAL OR COSMETIC COMPOSITIONS COMPRISING SAME

(84) Etats contractants désignés:
**AT BE CH DE DK ES FR GB GR IE IT LI LU MC NL PT SE**

(30) Priorité: **20.07.1994 FR 9408980**

(43) Date de publication de la demande:
**07.05.1997 Bulletin 1997/19**

(73) Titulaire:
**Pierre Fabre Dermo-Cosmetique**
**92100 Boulogne (FR)**

(72) Inventeurs:
- **LAGARDE, Isabelle**
  **F-31520 Ramonville-Saint-Agne (FR)**
- **COUTELLE, Valérie**
  **F-31680 Labarthe sur Leze (FR)**
- **NAVARRO, Roger**
  **F-09100 Pamiers (FR)**
- **JEANJEAN, Michel**
  **F-31320 Castanet-Tolosan (FR)**
- **GOORIS, Eric**
  **F-31450 Pompertuzat (FR)**

(74) Mandataire: **Ahner, Francis et al**
**CABINET REGIMBEAU**
**26, avenue Kléber**
**75116 Paris (FR)**

(56) Documents cités:
**EP-A- 0 070 525** **EP-A- 0 566 495**
**FR-A- 2 618 072**

- **POSTGRADUATE MEDICINE, vol. 91, no. 2, 1 Février 1992 pages 239-252, M. S. COHN 'Superficial fungal infections'**
- **DATABASE EMBASE (HOST STN) ELSEVIER SCIENCE PUBLISHERS, AMSTERDAM, NL AN=94239351, U. SCHRODER 'Hauterkrankungen durch Bakterien und Protozoen bei HIV-Infizierten.' & ALLERGOLOGIE, vol. 17, no. 7, 1994 pages 303-308,**

EP 0 771 187 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention se rapporte à de nouvelles compositions utiles en dermatologie et/ou cosmétologie présentant une activité antifongique améliorée.

Le traitement des infections fongiques cutanées est assez limité par manque de thérapeutiques. Les problèmes associés à ces pathologies, effets secondaires, absence de réponse au traitement ou développement de la résistance des germes, sont une réalité du terrain.

Parmi les affections cutanées provoquées par des champignons, on distingue celles dues aux levures et celles dues aux dermatophytes.

Les infections cutanées provoquées par les levures *Pityrosporum* ou *Candida* sont en recrudescence notamment du fait de l'augmentation des allergies cutanées et de celui du nombre de patients présentant un syndrome d'immunodéficience acquise.

En effet, les levures lipophiles *Pityrosporum* orbiculare et *Pityrosporum ovale* sont présentes en tant que saprophytes de la peau, mais lorsqu'elles se transforment sous leur forme active d'hyphe connue sous le nom de *Malassezia furfur*, on aboutit au pityriasis versicolor. Cette éruption très commune chez les jeunes adultes, se présente sous forme de petites surfaces circulaires avec desquamations blanches, rosées ou marrons. Les lésions se produisent au niveau du tronc, sur la partie proximale des bras ou des jambes et peuvent converger. La bonne implantation du germe est confirmée lorsqu'on assiste à une dépigmentation de la peau. Ceci est dû à la production d'un acide dicarboxylique, par la levure, qui résulte de l'inhibition de la tyrosinase. Cet effet, contraire à la synthèse de la mélanine, est inesthétique et constitue une des premières indications cliniques chez le patient, la colonisation cutanée par ce germe étant indolore.

Lors des traitements, il est essentiel que toutes les zones infestées soient inclues afin de s'assurer que le problème est éradiqué. Les patients soumis à une corticothérapie orale ou bien immunocompromis, peuvent développer une infestation massive. Lorsqu'une détection clinique n'est pas réalisée et que les stéroïdes topiques sont prescrits, une éruption accélérée peut être développée.

L'intérêt pour la dermatite séborrhéique a été relancé depuis la venue sur le marché du Kétoconazole, afin de traiter par voie orale et topique, les composantes inflammatoires de cette affection provoquée aussi par *Pityrosporum*. Ceci a mené à la reconnaissance d'une maladie écartée au départ mais reconnue maintenant comme le pityrosporum folliculitis. Sa manifestation principale est une folliculite du tronc de patients jeunes et moyennement âgés, et est fréquemment associée à la dermatite séborrhéique.

Cette dernière se manifeste sous forme d'éruptions avec des desquamations grisâtres du cuir chevelu, des oreilles, des plis inguinaux, du tronc et du dos.

Les desquamations au niveau des plis des paupières et des narines, sont aussi une manifestation de l'atteinte par cette levure.

Il est probable que de très nombreux facteurs externes jouent un rôle en altérant les effets saprophytes de *Pityrosporum* dans le pityriasis versicolor et la dermatite séborrhéique, ce qui induit la modification du comportement cutané de la levure.

Les autres pathologies à levures sont les candidoses superficielles cutanéomuqueuses. *Candida albicans* est l'agent pathogène le plus souvent retrouvé mais d'autres espèces peuvent aussi être présentes.

Comme les espèces de *Candida* se multiplient aisément dans une atmosphère chaude et humide, les candidoses superficielles siègent au niveau des plis axillaires ou adjacents aux orifices corporels. Des plaques érythémateuses et humides sont visibles et peuvent donner lieu à des pustules aux margelles des lésions. Bien entendu, des facteurs favorisant, tels que le traitement par antibiotique à large spectre, les stéroïdes systémiques et autres molécules immunosuppressives, favorisent l'émergence de candidoses bucco-digestives.

Certains diabètes, l'hypoparathyroïdisme peuvent avoir comme conséquence des paronychies chroniques et nécessiter un traitement topique complémentaire.

Enfin, dans le cas d'immunité déficiente, on assiste à une réaction granulomateuse comme dans les cas de candidose digestive. Lorsque les malades sont sévèrement immunocompromis, on assiste à une dissémination à la fois systémique et cutanée de la levure.

Mis à part les effets irritants cités, une réponse immunologique a été décrite avec ces levures. Elle est d'ordre humoral et cellulaire. De hauts taux d'anticorps sériques anti-pityrosporum lors de pellicules, ont été démontrés.

De plus, la dermatite séborrhéique est plus commune chez des patients présentant un terrain atopique, dermatite atopique cervico-céphalique, avec présence d'Ig E spécifiques antipityrosporum orbiculaire dont le taux est hautement corrélé avec la sévérité de la maladie. En ce qui concerne les dermatophytoses, nous pouvons citer le pied d'athlète, les teignes ainsi que toutes les onychomycoses.

Face à l'ensemble de ces pathologies, peu de thérapeutiques sont réellement efficaces.

Les imidazolés nécessitent au minimum trois semaines d'un régime biquotidien, les lésions cutanées étant souvent très étendues, et le patient manque souvent de produit afin de traiter l'ensemble des lésions corporelles (visibles

notamment en lumière de Wood par présence d'une couleur jaune clair). L'utilisation du shampooing au kétaconazole a bien été évoquée pour améliorer l'application mais le patient peut manquer son traitement lorsqu'une grande surface du dos est impliquée. De plus, les imidazolés sont fongistatiques et la probabilité de résistance est donc élevée. EP-0 070 525 décrit des compositions comprenant un imidazole (I) et une autre substance choisie dans un groupe, lui-même comprenant le crotamiton lequel est utilisé dans une quantité suffisante pour dissoudre le composé (I).

Les autres traitements locaux tels que les shampooings à base de pyrithione de zinc, de sulfure de sélénium ou de coaltar, montrent que la rémission n'est pas complète après environ un mois de traitement.

Il existe donc un réel besoin pour un produit antifongique qui aurait différentes qualités telles que l'efficacité, la rapidité d'action et présentant une excellente tolérance cutanée.

La présente invention concerne donc un nouveau produit de combinaison, dont l'association synergique présente une activité antifongique améliorée, actif sur des souches généralement résistantes aux antifongiques usuels, notamment aux imidazolés comme l'éconazole.

La présente invention concerne également une composition dermatologique et/ou cosmétique comprenant ladite association synergique de produits.

La synergie peut être mise en évidence par le calcul des Fic Index (Fractionnal inhibitory Concentration Index) ou FFC d'un produit A qui se définit comme suit :

$$FFCA = \frac{\text{CMF du produit en association}}{\text{CMF du produit A seul}}$$

CMF définissant la concentration minimale de fongicide pour laquelle on obtient une diminution de 4.Log 10 de l'inoculum ensemencé en 5 minutes de contact à 20°C.

L'association de deux antifongiques A et B est synergique si la somme des FFC (ou FFC Index) (FFCA + FFCB) est inférieure ou égale à 0,75.

Plus la valeur FFC Index est faible, plus la synergie est importante.

On considère qu'il y a simple additivité pour des valeurs d'Index comprises entre 0,75 et 1,1 et indifférence dans l'intervalle compris entre 1,1 et 2. Au-delà, l'association est considérée comme antagoniste.

Le nouveau produit selon l'invention consiste d'une part en un agent antifongique sélectionné parmi les 1-hydroxy-2-pyridones de formule générale I

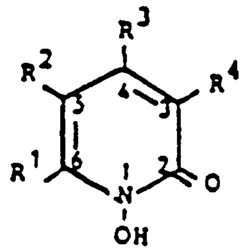

(I)

dans laquelle $R_1$ représente un reste hydrocarboné saturé ayant de 6 à 9 atomes de carbone,
l'un des restes $R_2$ et $R_4$ représente un atome d'hydrogène et l'autre représente un atome d'hydrogène ou le groupe méthyle ou éthyle, et
$R_3$ représente un reste alkyle ayant 1 ou 2 atomes de carbone, et leurs sels physiologiquement acceptables,
et d'autre part, du crotamiton, comme potentialisateur de l'activité antifongique de la 1-hydroxy-2-pyridone.

La composition dermatologique et/ou cosmétique selon l'invention contient pour sa part une association synergique de la 1-hydroxy-2-pyridone de formule générale I, telle que définie ci-dessus, et du crotamiton, avec au moins un excipient pharmaceutiquement acceptable.

Par saturé, on entendra un reste hydrocarboné ne contenant pas de liaisons multiples aliphatiques, telles que des liaisons éthyléniques ou acétyléniques.

De préférence, R1 est un reste alkyle ou cycloalkyle, et dans ce cas un reste cyclohexyle qui peut être lié au noyau pyridone par un alkylène, ou encore être substitué par un groupe alkyle.

R1 peut également représenter un radical aromatique ou un radical aromatique lié au noyau pyridone par un reste alkylène.

Le radical aromatique est avantageusement un groupe phényle, éventuellement subsitué par un ou plusieurs groupes alkyles.

Parmi les composés de formule générale I utiles selon la présente invention, on citera en particulier la 1-hydroxy-4-méthyl-6-n-hexyl-, -6-iso-hexyl-, -6-n-heptyl- ou -6-iso-heptyl-2-pyridone, la 1-hydroxy-4-méthyl-6-octoyl- ou -6-iso-octyl-2-pyridone, en particulier sous forme de 1-hydroxy-4-méthyl-6-(2,4,4-triméthylpentyl)-hydroxy-4-méthyl-6-cyclohexyl-2-pyridone, la 1-hydroxy-4-méthyl-6-cyclo-hexylméthyl- ou -6-cyclohexyléthyl-2-pyridone, le reste cyclohexyle pouvant dans chaque cas porter encore un radical méthyle, la 1-hydroxy-4-méthyl-6-(2-bicyclo[2.2.1]heptyl)-2-pyridone, la 1-hydroxy-3,4-diméthyl-6-benzyl- ou -6-diméthylbenzyl-2-pyridone et la 1-hydroxy-4-méthyl-6-(β-phényl-éthyl)-2-pyridone.

D'une manière préférentielle, la 1-hydroxy-2-pyridone est du ciclopirox (R1=cyclohexyl, R2=R4=H et R3=CH3) ou de l'octopirox (R1=2,4,4-triméthylpentyl, R2=R4=H et R3=CH3) ainsi que leurs sels physiologiquement acceptables, notamment leur sel d'éthanolamine.

L'intérêt des 1-hydroxy-2-pyridones réside dans leur action, au niveau du métabolisme protéique des levures ; c'est-à-dire après avoir pénétré dans la cellule et non au niveau de la synthèse de l'ergostérol, comme les imidazolés qui agissent au niveau pariétal.

Le crotamiton, ou N-éthyl-N-O-tolylcrotonamide a pour sa part été décrit comme scabicide, antiprurigineux et comme antifongique.

D'une manière avantageuse, le rapport pondéral 1-hydroxy-2-pyridone/crotamiton dans le produit de combinaison selon l'invention est compris entre 4/1 et 1/4, de préférence voisin de 1.

Les constituants du nouveau produit de combinaison selon l'invention sont destinés à être employés simultanément.

Toutefois, ils pourront également être employés en association séparément ou encore décalés dans le temps.

Un même rapport pondéral préférentiel entre la 1-hydroxy-2-pyridone de formule I, telle que définie ci-dessus et le crotamiton, sera recherché dans les compositions dermatologiques et/ou cosmétiques selon l'invention.

Selon cet aspect de la présente invention, l'association synergique telle que définie plus haut sera présente dans la composition à une teneur comprise entre 0,5 et 4 % en poids.

Les compositions sont de préférence sous la forme de shampooing, de lotion ou encore de solution aérosol.

Les exemples qui suivent sont destinés à illustrer l'invention, sans aucunement en imiter la portée.

Dans ces exemples, on se référera aux tableaux en annexe qui résument l'activité fongicide des produits seuls et associés sur *Pityrosporium ovale* sauvage résistant à l'éconazole, et *Candida albicans* ATCC 9021.

**EXEMPLE 1 : Association ciclopiroxolamine/crotamiton**

Des souches de *Malassezia furfur* (*Pityrosporum ovale*) ont été testées pour leur sensibilité vis-à-vis des principes actifs seuls ou bien en association selon l'invention.

Les souches sont issues du Laboratoire de Parasitologie du Centre Hospitalier Régional de Rangueil de TOULOUSE (France) et une souche est éconazole résistante (éco-R). Elles ont été cultivées lors des essais sur milieu Dixon solide.

La technique utilisée est originale car elle utilise à la fois le principe de la technique de l'échiquer et celui de la norme Afnor par filtration sur membrane relative à la fongicidie avec un temps de contact germe-molécules de 5 minutes.

C'est une macro-méthode qui a donc été réalisée ici avec validations à chaque essai du témoin solvant des molécules (Tween® 20 %, éthanol 17 % dans l'eau distillée).

Ce solvant inhibe légèrement l'activité de la ciclopiroxolamine, et il faut donc s'attendre à de meilleurs résultats en pratique. Les autres témoins sont celui de la vigueur de la souche et de l'efficacité des produits seuls, soit :

- ciclopiroxolamine 4 %
- crotamiton 4 %

Le tableau 1 représente les moyennes géométiques de la réduction logarithmique sur deux *Pityrosporium ovale* sauvages dont une éco-R, le nombre d'essais ainsi que le pourcentage d'efficacité.

La norme Afnor considère qu'il y a fongicidie pour une réduction de 4 Log 10 de l'inoculum après 15 minutes de

contact.

Nous avons donc ici en cinq minutes de contact 100 % d'activité pour :

- Crotamiton 4 % (produit utilisé seul)
-

| | | |
|---|---|---|
| Crotamiton 2 % | ) | |
| | ) | (association synergique) |
| Ciclopiroxolamine 2 % | ) | |

et 50% d'activité pour

- Ciclopiroxolamine 4 % (produit utilisé seul)

Pour des raisons techniques de solubilisation de la molécule, nous avons considéré que les concentrations supérieures en ciclopiroxolamine tendraient vers les 100 % d'activité à environ 6 % de ciclopiroxolamine. Le FFC Index est donc inférieur à 0,7.

Le pH de l'ensemble de ces études se situe autour de 8.

**<u>EXEMPLE 2</u> : Association octopiroxolamine/crotamiton**

L'association de l'octopirox avec le crotamiton a été testé selon la méthodologie de l'exemple 1 sur une souche *Pityrosporium ovale* éco-R et *C. albicans* ATCC 9021.

Le tableau 2 représente les résultats obtenus sur *P. ovale* éco-R après cinq minutes de contact pour l'association :

- crotamiton 1 % - octopirox 1 % pour lequel nous avons 100 % d'efficacité
- alors que les produits seuls à double ou quadruple concentrations sont moins actifs.

En effet, le crotamiton à 4 % donne une efficacité de 84 % et l'octopirox à 2 %, une efficacité de 45 %.

Nous voyons donc que le rapport des concentrations nous donne un FFC Index inférieur à 0,75.

Le même solvant a été utilisé et validé à chaque essai comme le spécifie la norme Afnor.

Ces Résultats ont été confirmés sur une levure de référence Afnor (*C.albicans* ATCC 9021). Les résultats sont reportés dans le tableau 3.

Les essais de l'exemple 2 ont été réalisés avec un pH plus basique = 9.

**TABLEAU 1**

| Produits concentrations (%) (m/v) | CPO(4) | CPO(2) | CPO(2) Crota (0,5) | CPO(2) Crota (1) | CPO(2) Crota (2) | Crota (1) | Crota (2) | Crota (4) |
|---|---|---|---|---|---|---|---|---|
| Mg | 2,05 | 0,43 | 1,11 | 2,90 | 4,09 | 0,03 | 0,77 | 3,77 |
| n | 7 | 7 | 3 | 7 | 3 | 7 | 7 | 3 |
| % activité | 50% | 10% | 25% | 75% | 100% | 1% | 20% | 100% |

CPO : ciclopirox olamine

Crota : crotamiton

Mg : moyenne géométrique du Log 10 de la réduction du nombre de germes

n : nombre d'essais indépendants

On voit une synergie pour CPO 2% / Crota 2% : les produits seuls, à double concentration CPO 4% ou Crota 4%, sont moins actifs que les produits associés moins concentrés.

TABLEAU 2

| Produits (%) | Octo (2) | Octo (1) Crota (1) | Crota (2) | Crota (4) |
|---|---|---|---|---|
| Mg<br>n | 1,85<br>2 | 4,07<br>2 | 1,59<br>2 | 3,26<br>2 |
| % activité | 45% | 100% | 40% | 83% |
| Octo: octopirox | | | | |

TABLEAU 3

| Produits (%) | Octo (2) | Octo (1) | Octo (1) Crota (1) | Octo (0,5) Crota (1) | Octo (0,5) Crota (1) | Crota (2) | Crota (4) |
|---|---|---|---|---|---|---|---|
| Mg<br>n | 3,18<br>3 | 0,36<br>3 | 4,94<br>3 | 0,57<br>2 | 2,54<br>3 | 2,43<br>3 | 4,95<br>3 |
| % activité | 60% | 7% | 100% | 10% | 50% | 50% | 100% |

**EXEMPLE 3 : Shampooing**

| | | |
|---|---|---|
| Octopiroxolamine | | ) |
| ou | | ) 0,5 à 2 % |
| Ciclopiroxolamine | | ) |
| Crotamiton | | 0,5 à 2 % |
| Décylglucoside (sol. 55 %) | | 10 g |
| Disodium cocoamphodiacetate (Sol. 38 %) | | 15 g |
| Cocamidopropyl bétaine (Sol. 30 %) | | |
| Cocamide MEA | | 5 g |
| Propylèneglycol | | 2,5 g |
| Parfum, colorant | | |
| Agent complexant | | |
| Eau déminéralisée | QSP | 100 ml |
| pH ajusté entre 7 et 9 | | |

**EXEMPLE 4 : Shampooing**

| | | |
|---|---|---|
| Octopiroxolamine | | ) |
| ou | | ) 0,5 à 2 % |
| Ciclopiroxolamine | | ) |
| Crotamiton | | 0,5 à 2 % |
| Laurylpolyglucose (sol. 50 %) | | 10 g |
| Disodium cocoamphodiproponate (Sol. 40 %) | | 8 g |
| Polysorbate 20 | | 1 à 3 g |
| Hydrogenated talloweth 60 | | |
| Myristyl glycol | | 2 à 3 g |
| N-hydroxytehyl-acétamide (Sol. 70 %) | | 0,5-1,5 % |
| Parfum complexant | | |
| Agent opacifiant | | |
| Eau déminéralisée | QSP | 100 ml |
| pH ajusté entre 7 et 9 | | |

**EXEMPLE 5 : Shampooing**

| | | |
|---|---|---|
| Octopiroxolamine | | ) |
| ou | | ) 0,5 à 2 % |
| Ciclopiroxolamine | | ) |
| Crotamiton | | 0,5 à 2 % |
| Décylglucoside (sol. 55 %) | | 6 g |
| Disodium cocoamphodiacetate (Sol. 38 %) | | |
| Cocamidopropyl diméthylamino Hydroxypropyl | | |
| Hydrolyse collagène (Sol. 30 %) 7 g | | |
| Cocamide DEA | | 3 à 5 g |
| Glycérine | | 2 g |
| Parfum, colorant | | |
| Eau déminéralisée | QSP | 100 ml |
| pH ajusté entre 7 et 9 | | |

**EXEMPLE 6 : Shampooing**

| | | |
|---|---|---|
| Ciclopiroxolamine ou octopiroxolamine | | ) |
| | | ) 0,5 à 2 % |
| Crotamiton | | ) |
| Alkyl éther sulfate de triéthanolamide | | |
| (Sol. à 30 %) | | 20 à 50 % |
| Dihydroxyéthanolamide d'acides gras | | |
| de Coprah | | |
| Ethylène diamine disodique | | 0,15 % |
| Chlorure de sodium (qs viscosité) | | 1 % |
| Parfum | | |
| Eau purifiée | QSP | 100 g |

Il est important de s'assurer que le pH de ces shampooings soit ajusté aux environs de 7-9 pour des raisons d'efficacité et de meilleure solubilisation des actifs. Il est bien évident que ces formules ne sont pas limitatives et qu'il est important de privilégier la compatibilité des tensio-actifs avec l'association 1-hydroxy-2-pyridone/crotamiton selon l'invention.

**EXEMPLE 7 : Lotion capillaire**

| | | |
|---|---|---|
| Octopiroxolamine | | ) |
| ou | | ) 0,5 à 2 % |
| Ciclopiroxolamine | | ) |
| Crotamiton | | 0,5 à 2 % |
| Chlorure de laurylpyridinium | | 0,01 à 0,100 |
| Diméthicone copolyol | | 0,10 à 0,50 % |
| Parfum | QS | |
| Mélange eau-alcool 30 % à 60 % Vol. | QSP | 100 ml |

**EXEMPLE 8 : Solution aérosol**

| | | |
|---|---|---|
| Octopiroxolamine | | ) |
| ou | | ) 0,5 à 2 % |
| Ciclopiroxolamine | | ) |
| Crotamiton | | 0,5 à 2 % |
| Cyclomethicone | | 1 à 5 % |
| Méthylol | | 30 ml |
| N-hydroxyéthylacetamide 70 % | | 1 à 5 % |
| Alcool éthylique | | 50 ml |
| Eau déminéralisée | QSP | 100 ml |

Azote QSP 9 bars pour pressurisation en boitier aérosol.

**Revendications**

1. Produit de combinaison caractérisé en ce qu'il consiste en l'association d'une part en un agent antifongique sélectionné parmi les 1-hydroxy-2-pyridones de formule générale I :

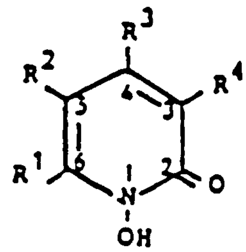

(I)

dans laquelle $R_1$ représente un reste hydrocarboné saturé ayant de 6 à 9 atomes de carbone,
l'un des restes $R_2$ et $R_4$ représente un atome d'hydrogène et l'autre représente un atome d'hydrogène ou le groupe méthyle ou éthyle, et $R_3$ représente un reste alkyle ayant 1 ou 2 atomes de carbone,
et leurs sels physiologiquement acceptables,
et d'autre part, du crotamiton, comme potentialisateur de l'activité de l'agent anti-fongique.

2. Produit selon la revendication 1, caractérisé en ce que dans la formule générale I, $R_1$ est un reste alkyle ou cycloalkyle, ledit cycloalkyle pouvant être lié au noyau pyridone par un alkylène, ou encore substitué par un groupe alkyle.

3. Produit selon la revendication 1, caractérisé en ce que dans la formule générale I, $R_1$ est un radical aromatique, éventuellement substitué par un ou plusieurs groupes alkyles, qui peut être lié au noyau pyridone par un reste alkylène.

4. Produit selon la revendication 1, caractérisé en ce que l'agent antifongique de formule générale I est du ciclopirox ou de l'octopirox, ainsi que leurs sels physiologiquement acceptables, notamment leur sel d'éthanolamine.

5. Produit selon l'une des revendications 1 à 4, caractérise en ce que le rapport pondéral 1-hydroxy-2-pyridone/crotamiton est compris entre 4/1 et 1/4, de préférence voisin de 1.

6. Produit contenant un agent antifongique sélectionné parmi les 1-hydroxy-2-pyridones de formule générale I, telles que définies dans les revendications 1 à 4, et du crotamiton, comme produit de combinaison pour une utilisation simultanée, séparée ou étalée dans le temps pour le traitement des infections fongiques cutanées.

7. Composition dermatologique et/ou cosmétique caractérisée en ce qu'elle contient l'association synergique d'une 1-hydroxy-2-pyridone et de crotamiton, telle que définie dans les revendications 1 à 5, et au moins un excipient pharmaceutiquement acceptable.

8. Composition selon la revendication 7, caractérisée en ce qu'elle contient entre 0,5 et 4 % en poids de l'association synergique.

## Claims

1. Combination product, characterized in that it consists of a combination of, on the one hand, an antifungal agent selected from 1-hydroxy-2-pyridones of general formula I:

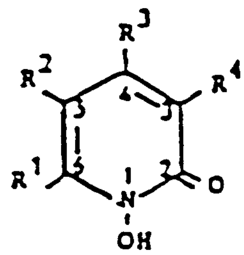

(I)

in which $R_1$ represents a saturated hydrocarbon-based residue having from 6 to 9 carbon atoms,
one of the residues $R_2$ and $R_4$ represents a hydrogen atom and the other represents a hydrogen atom or a methyl or ethyl group, and $R_3$ represents an alkyl residue having 1 or 2 carbon atoms,
and their physiologically acceptable salts,
and, on the other hand, crotamiton, as potentiator of the activity of the antifungal agent.

2. Product according to Claim 1, characterized in that, in the general formula I, $R_1$ is an alkyl or cycloalkyl residue, it being possible for the said cycloalkyl residue to be linked to the pyridone ring via an alkylene, or alternatively to be substituted with an alkyl group.

3. Product according to Claim 1, characterized in that, in the general formula I, $R_1$ is an aromatic radical, optionally substituted with one or more alkyl groups, which can be linked to the pyridone ring via an alkylene residue.

4. Product according to Claim 1, characterized in that the antifungal agent of general formula I is ciclopirox or octopirox, as well as their physiologically acceptable salts, in particular their ethanolamine salt.

5. Product according to one of Claims 1 to 4, characterized in that the 1-hydroxy-2-pyridone/crotamiton weight ratio is between 4/1 and 1/4, preferably close to 1.

6. Product containing an antifungal agent selected from the 1-hydroxy-2-pyridones of general formula I, as defined in Claims 1 to 4, and crotamiton, as a combination product for a simultaneous or separate use, or for use spread out over time, for the treatment of fungal skin infections.

7. Dermatological and/or cosmetic composition, characterized in that it contains a synergistic combination of a 1-hydroxy-2-pyridone and crotamiton, as defined in Claims 1 to 5, and at least one pharmaceutically acceptable excipient.

8. Composition according to Claim 7, characterized in that it contains between 0.5 and 4% by weight of the synergistic combination.

## Patentansprüche

1. Kombinationsprodukt, dadurch charakterisiert, daß es aus einer Kombination von einerseits einem antimykotischen Mittel besteht, das unter den 1-Hydroxy-2-pyridonen der allgemeinen Formel I ausgewählt wird:

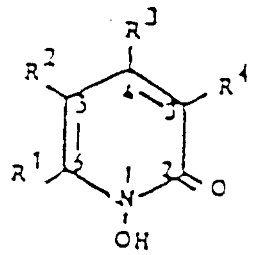

(I)

wobei $R_1$ einen gesättigten Kohlenwasserstoffrest mit 6 bis 9 Kohlenstoffatomen darstellt, einer der Reste $R_2$ und $R_4$ ein Wasserstoffatom darstellt und der andere ein Wasserstoffatom oder eine Methyl- oder Ethylgruppe darstellt, und $R_3$ einen Alkylrest mit 1 oder 2 Kohlenstoffatomen darstellt, und ihren physiologisch verträglichen Salzen, und andererseits von Crotamiton als Aktivitätsverstärker der antimykotischen Mittels.

2. Produkt nach Anspruch 1, dadurch charakterisiert, daß in der allgemeinen Formel I $R_1$ ein Alkyl- oder Cycloalkylrest ist, wobei der Cycloalkylrest durch ein Alkylen an den Pyridon-Kern gebunden oder durch eine Alkylgruppe substituiert sein kann.

3. Produkt nach Anspruch 1, dadurch charakterisiert, daß in der allgemeinen Formel I R ein aromatischer Rest ist, gegebenenfalls substituiert durch eine oder mehrere Alkylgruppen, der an den Pyridon-Kern durch einen Alkylenrest gebunden sein kann.

4. Produkt nach Anspruch 1, dadurch charakterisiert, daß das antimykotische Mittel der allgemeinen Formel I Ciclopirox oder Octopirox ist, oder deren physiologisch verträgliche Salze, insbesondere ihr Ethanolamin-Salz.

5. Produkt nach einem der Ansprüche 1 bis 4, dadurch charakterisiert, daß das Gewichtsverhältnis 1-Hydroxy-2-pyridon/Crotamiton zwischen 4/1 und 1/4 liegt, bevorzugt in der Nähe von 1.

6. Produkt, das ein unter den 1-Hydroxy-2-pyridonen der allgemeinen Formel I, wie in den Ansprüchen 1 bis 4 definiert, ausgewähltes antimykotisches Mittel und Crotamiton enthält, als Kombinationsprodukt zur gleichzeitigen, getrennten oder zeitlich aufeinanderfolgenden Verwendung für die Behandlung von Haut-Pilzinfektionen.

7. Dermatologische und/oder kosmetische Zusammensetzung, dadurch charakterisiert, daß sie die synergistische Kombination eines 1-Hydroxy-2-pyridons und von Crotamiton enthält wie in den Ansprüchen 1 bis 5 definiert, und mindestens einen pharmazeutisch verträglichen Exzipienten.

8. Zusammensetzung nach Anspruch 7, dadurch charakterisiert, daß sie zwischen 0,5 und 4 Gew.-% der synergistischen Kombination enthält.